(19) Europäisches Patentamt
European Patent Office
Office européen des brevets

(11) **EP 2 092 930 B1**

(12) **EUROPÄISCHE PATENTSCHRIFT**

(45) Veröffentlichungstag und Bekanntmachung des Hinweises auf die Patenterteilung:
**04.01.2017 Patentblatt 2017/01**

(51) Int Cl.:
*A61K 8/37* (2006.01)   *A61K 8/27* (2006.01)
*A61K 8/29* (2006.01)   *A61K 8/35* (2006.01)
*A61K 8/41* (2006.01)   *A61K 8/49* (2006.01)
*A61Q 17/04* (2006.01)   *A61K 8/06* (2006.01)

(21) Anmeldenummer: **08003247.7**

(22) Anmeldetag: **22.02.2008**

(54) **Lichtschutzzubereitung in Form einer O/W-Emulsion mit einem Lichtschutzfaktor von _> 50**

Sun block preparation in the form of an o/w emulsion with a sun block factor of _> 50

Préparation de protection contre la lumière sous la forme d'émulsions huile/eau ayant un facteur de protection contre la lumière de _> 50

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**26.08.2009 Patentblatt 2009/35**

(73) Patentinhaber: **STADA ARZNEIMITTEL AG 61118 Bad Vilbel (DE)**

(72) Erfinder:
• **HANSEN, Peter, Dr. 63303 Dreieich (DE)**
• **HEPPNER, Andrea 61197 Florstadt (DE)**
• **RILLMANN, Thomas, Dr. 76756 Bellheim (DE)**

• **SCHUMANN, Christof 35767 Breitscheid-Erdbach (DE)**

(74) Vertreter: **Hamm & Wittkopp Patentanwälte PartmbB Jungfernstieg 38 20354 Hamburg (DE)**

(56) Entgegenhaltungen:
**EP-A- 1 281 390       WO-A-2006/034985
WO-A-2006/035000     DE-A1- 10 138 499
DE-A1- 10 141 473     DE-A1- 10 155 716**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft Lichtschutzzubereitungen in Form einer O/W-Emulsion, die einen Lichtschutzfaktor von ≥ 50 aufweisen und als Filtersubstanzen die pigmentären UV-Filter Titandioxid, Zinkoxid und Methylene Bis-Benzotriazolyl Tetramethylbutylphenol und einen weiteren UV-A Filter enthalten, wie in Anspruch 1 definiert.

Die schädigende Wirkung des ultravioletten Teils der Sonnenstrahlung auf die Haut ist seit langem bekannt. Derartige Schädigungen können eine einfache Hautreizung, z.B. ein leichter Sonnenbrand sein, können sich aber auch in Zellschädigungen manifestieren. Es ist bekannt, dass vor allem durch UV-B-Strahlung (das ist Strahlung mit einer Wellenlänge zwischen 290 nm und 320 nm) in der Haut DNS-Schäden hervorgerufen werden können, die zu Zellmutationen und somit zu Hautkrebs führen.

Auch werden die vorzeitige Hautalterung, die Bildung von Falten und die Erschlaffung des Hautbindegewebes durch UV-Strahlung beschleunigt; verantwortlich hierfür ist vor allem die langwellige UV-A-Strahlung (das ist Strahlung mit einer Wellenlänge zwischen 320 nm und 400 nm). Auch sie begünstigt die Auslösung einer Erythembildung oder verstärkt diese Reaktion bei manchen Personen, und sie kann sogar die Ursache für durch Licht ausgelöste toxische oder allergische Reaktionen sein.

Zur Vorbeugung gegen derartige Schädigungen sind in den letzten Jahren immer effektivere Sonnenschutzmittel entwickelt worden, in denen zahlreiche physikalische und chemische Filtersubstanzen die Haut vor UV-A-Strahlung und vor UV-B-Strahlung schützen sollen.

Physikalische Lichtschutzfilter, wie z.B. Oxide von Metallen, wirken auf der Haut verteilt wie kleine Spiegel, die die UV-Strahlung reflektieren und streuen.

Durch chemische Lichtschutzfilter wird die auf die Haut auftreffende UV-Strahlung in Wärmeenergie umgewandelt. Chemische Lichtschutzfilter können sowohl lipophile als auch hydrophile Eigenschaften aufweisen und entsprechend dieser Eigenschaften unterscheidet man zwischen öllöslichen und wasserlöslichen Filtern.

Lichtschutzzubereitungen werden oftmals als Emulsionen, Lotionen oder Gele formuliert.

Aufgabe der vorliegenden Erfindung war es, gegenüber dem Stand der Technik verbesserte Lichtzusammensetzungen anzugeben.

Insbesondere war es Aufgabe der vorliegenden Erfindung, Lichtschutzzusammensetzungen anzugeben, die als O/W-Emulsion formuliert, einen Lichtschutzfaktor von ≥ 50 aufweisen.

Durch umfangreiche Untersuchungen hat die Anmelderin in unerwarteter und überraschender Weise festgestellt, dass eine Kombination der pigmentären UV-Filter Titandioxid, Zinkoxid und Methylene Bis-Benzotriazolyl Tetramethylbutylphenol  (2,2'-Methylen-bis(6-(2H-benzotriazol-2-yl)-4-(1,1,3,3-tetramethylbutyl)phenol)  die Erzielung von Sonnenschutzzusammensetzungen erlaubt, die als O/W-Emulsion formuliert, einen deutlich verbesserten UV-Schutz aufweisen, der bei einem Lichtschutzfaktor von ≥ 50 liegt. Sie hat des Weiteren überraschend gefunden, dass stabile Formulierungen mit derartig hohen Lichtschutzfaktorwerten von O/W-Emulsionen durch Einarbeitung der pigmentären UV-Filter Titandoxid, Zinkoxid und Methylene Bis-Benzotriazolyl Tetramethylbutylphenol auch erzielt werden können, wenn keine PEG-Emulgatoren in diese Zusammensetzungen eingearbeitet werden.

Gegenstand der vorliegenden Erfindung ist zum einen eine Lichtschutzzubereitung, enthaltend die pigmentären UV-Filter Titandioxid, Zinkoxid und Methylene Bis-Benzotriazolyl Tetramethylbutylphenol. In einer bevorzugten Ausführungsform enthält die erfindungsgemäße Lichtschutzzubereitung keine PEG-Emulgatoren.

Weiterhin bevorzugt enthält die erfindungsgemäße Lichtschutzzubereitung zusätzlich mindestens einen UV-A-Filter, bevorzugt Diethylamino Hydroxybenzoyl Hexyl Benzoate (Uvinul A plus der Firm BASF) und/oder Butyl Methoxydibenzoylmethane (Parsol 1789 von DSM bzw. Neo Heliopan 357 von Symrise). Es wurde überraschend gefunden, dass die Verwendung der reinen UV-A-Filter Butyl Methoxydibenzoylmethane und/oder Diethylamino Hydroxybenzoyl Hexyl Benzoate zu einer Steigerung der UV-B-Schutzwirkung der vorliegenden Lichtschutzzubereitungen mit den pigmentären UV-Filtern führt. Erfindungsgemäß bevorzugte PEG-Emulgator-freie O/W-Emulsionen können somit einmal eine Kombination der pigmentären UV-Filter Titandioxid, Zinkoxid und Methylene Bis-Benzotriazolyl Tetramethylbutylphenol mit Diethylamino Hydroxybenzoyl Hexyl Benzoate und ein andermal eine Kombination der pigmentären UV-Filter Titandioxid, Zinkoxid und Methylene Bis-Benzotriazolyl Tetramethylbutylphenol mit Butyl Methoxydibenzoylmethane enthalten.

In weiterhin bevorzugten Ausführungsformen enthalten die erfindungsgemäßen Zusammensetzungen zusätzlich weitere lösliche UV-A- oder UV-B-Filter, insbesondere das Ethylhexyl Methoxycinnamate.

O/W-Emulsionen stellen eine übliche Formulierungsgrundlage für Sonnenschutzzusammensetzungen dar. Sie lassen sich gut auf die Haut auftragen und ziehen schnell ein. Mitverantwortlich dafür ist der Emulgator. Oft wurden im Stand der Technik zur Herstellung von O/W-Emulsionen, insbesondere mit höheren Lichtschutzfaktorwerten, PEG-Emulgatoren verwendet, die aber nachteilige Hautreaktionen auslösen können. Es war bisher nicht möglich, stabile O/W-Emulsionen mit Lichtschutzfaktoren von ≥ 50 herzustellen, die keine PEG-Emulgatoren enthalten.

Der Lichtschutzfaktor ist die Maßzahl, mit der die Wirksamkeit einer Lichtschutzformulierung gemessen wird und gibt an, wie viel länger man sich mit der auf die Haut aufgetragenen Lichtschutzformulierung der Sonne aussetzen kann, als dies mit der jeweils individuellen Eigenschutzzeit möglich wäre, bis ein Sonnenbrand entsteht. Wird vor und nach Auftragen eines Lichtschutzpräparates die minimale Erythemdosis (MED), das heißt die Menge an UV-B-Bestrahlung, die ein gerade erkennbares Erythem induziert, bestimmt, ergibt sich der Lichtschutzfaktor (LSF) nach folgender Formel:

$$\text{LSF} = (\text{MED mit Sonnenschutzmittel}) / (\text{MED ohne Sonnenschutzmittel})$$

Der Lichtschutzfaktor wird nach der "International Sun Protection Factor Method (2006)" bestimmt.

Die erfindungs gemäßen Titandioxidpigmente (TiO$_2$) werden vorzugsweise in mikronisierter Form eingesetzt, vorzugsweise in mittleren Teilchengrößen < 100 $\mu$m, besonders bevorzugt in mittleren Teilchengrößen zwischen 10 nm und 200 nm und besonders bevorzugt in mittleren Teilchengrößen zwischen 10 nm und 100 nm. Dabei ist es besonders vorteilhaft, wenn die Titandioxidpigmente in hydrophober Form vorliegen, das heißt, dass sie oberflächig wasserabweisend ausgestaltet sind. Dies kann z. B. dadurch erfolgen, dass die Pigmentteilchen mit einer hydrophoben Oberflächenschicht aus Silikon überzogen werden. Derartige Pigmente sind kommerziell erhältlich beispielsweise von der Firma Tayca unter der Handelsbezeichnung MT 100 T, MT 100 Z oder von der Firma Degussa unter der Bezeichnung T 805 sowie von der Firma Nordmann, Rassmann unter der Bezeichnung MT 100 Z.

Die erfindungsgemäßen Titandioxidpigmente werden bevorzugt in Mengen 3 bis 12 Gew.-% eingesetzt.

Die erfindungsgemäßen Zinkoxide werden ebenfalls vorzugsweise in mikronisierter Form eingesetzt, vorzugsweise in mittleren Teilchengrößen < 100 $\mu$m, besonders bevorzugt in mittleren Teilchengrößen < 300 nm, wie z. B. Z-Cote HP1 von der BASF oder Zinkoxid NDM von Symrise.

Die erfindungsgemäßen Zinkoxide werden bevorzugt in Mengen von 0,2 bis 2 Gew.-% eingesetzt.

Das Methylene Bis-Benzotriazolyl Tetramethylbutylphenol (2,2'-Methylen-bis(6-(2H-benzotriazol-2-yl)-4-(1,1,3,3-tetramethylbutyl)phenol) wird ebenfalls vorzugsweise in mikronisierter Form, besonders bevorzugt in einer Teilchengröße von < 100 $\mu$m, insbesondere von etwa 0,2 $\mu$m eingesetzt und ist beispielsweise als 50 %-ige Dispersion von der Firma Ciba Spezialitätenchemie unter der Handelsbezeichnung Tinosorb M erhältlich.

Das Methylene Bis-Benzotriazolyl Tetramethylbutylphenol wird vorzugweise in Mengen von 5 bis 8 Gew.-% in den erfindungsgemäßen Zusammensetzungen eingesetzt.

Vorzugsweise liegt das Verhältnis von anorganischen zu organischen Mikropigmenten in den erfindungsgemäßen Zusammensetzungen im Bereich von 0,2 bis 5, insbesondere von 0,5 bis 2.

Das Verhältnis des pigmentären Zinkoxids zum pigmentären Titandioxid liegt in den erfindungsgemäßen Zusammensetzungen vorzugsweise im Bereich von 0,01 bis 1, insbesondere von 0,05 bis 0,5.

Die Menge an UV-A-Filtern, insbesondere Diethylamino Hydroxybenzoyl Hexyl Benzoate und/oder Butyl Methoxydibenzoylmethane, beträgt in den erfindungsgemäßen Zubereitungen vorzugsweise 0,5 bis 8 Gew.-%.

Die erfindungsgemäßen Lichtschutzzusammensetzungen können des Weiteren einen und/oder mehrere weitere organische UV-Filter enthalten, die öllösliche oder wasserlösliche UV-A- und/oder UV-B-Filter sind. Dabei sind als geeignete organische UV-Filter insbesondere die folgenden zu nennen:

p-Aminobenzoesäurederivate, z.B. 4-Aminobenzoesäure, 4-Dimethylaminobenzoesäure-2-ethylhexylester (Eusolex 6007) oder ethoxyliertes Ethyl-4-aminobenzoat (Uvinul P25),

Salicylsäurederivate, z.B. 3,3,5-Trimethyl-cyclohexyl-salicylat (Neo Heliopan HMS) oder Salicylsäure-2-ethylhexylester (Neo Heliopan OS),

Benzophenonderivate, z.B. 2-Hydroxy-4-methoxy-benzophenon (Neo Heliopan BB), 2-Hydroxy-4-methoxy-benzophenon-5-sulfonsäure und ihr Natriumsalz (Uvinul MS 40),

Sulfonsäurederivate, z.B. 2-Phenylbenzimidazol-5-sulfonsäure und ihre Kalium-, Natrium- und Triethanolaminsalze (Neo Heliopan Hydro) oder Benzol-1,4-di(3-methyliden-10-camphersulfonsäure) (Mexoryl SX),

Dibenzoylmethenderivate,

Diphenylacrylate, z.B. 2-Cyano-3,3-diphenylacrylsäure-2-ethylhexylester (Neo Heliopan 303),

Methoxyzimtsäurederivate, z.B. 4-Methoxyzimtsäure-2-ethylhexylester (Neo Heliopan AV) oder 4-Methoxy-zimtsäureisoamylester (Neo Heliopan E 1000),

Campherderivate, z.B. 3·(4'-Trimethylammonium)-benzyliden-bornan-2-onmethylsulfat (Mexoryl SO), 3-(4'-Sulfo)-benzyliden-bornan-2-on und seine Salze (Mexoryl SL) oder 3-(4'-Methylbenzyliden)-DL-campher (Eusolex 6300) oder 3-Benzylidencampher (Mexoryl SDS20),

Benzotriazolderivate, z.B, 2-(2H-Benzotriazol-2-yl)-4-methyl-6-(2-methyl-3-(1,3,3,3-tetramethyl-1-(trimethylsilyloxy)disiloxanyl)propyl)phenol (Mexoryl XL),

Benzimidazolderivate, z.B. 2,2'-(1,4-Phenylen)bis(1H-benzimidazol-4,6-disulfonsäure, Mononatriumsalz) (Neo Heliopan AP),

Benzoxazol-Derivate, z.B. 2,4-Bis-[5-1(dimethylpropyl)benzoxazol-2-yl-(4-phenyl)-imino]-6-(2-ethylhexyl)-imino-1,3,5-triazin (Uvasorb K2A),

Polymer von N-[2(und 4)-(2-oxoborn-3-ylidenmethyl)benzyl]acrylamid (Mexoryl SW),

3-(4-(2,2-bis Ethoxycarbonylvinyl)-phenoxy)propenyl)-methoxysiloxan/Dimethylsiloxan-copolymer (Parsol SLX).

Diese organischen UV-Filter sind, sofern enthalten, bevorzugt jeweils in Mengen von 0,1 bis 10 Gew.-%, vorzugsweise 0,5 bis 8 Gew.-%, insbesondere 0,1 bis 7 Gew.% in den erfindungsgemäßen Zusammensetzungen enthalten.

Vorzugsweise sind die erfindungsgemäßen Lichtschutzzubereitungen frei von UV-Filtern vom Triazintyp, d.h. von Triazinderivaten, wie z.B. 2,4,6-Tris[p-(2-ethylhexyl-oxycarbonyl)anilino] 1,3,5-triazin (Uvinul T 150), 4,4'-[(6-[4-((1,1·Dimethylethyl)aminocarbonyl)phenyl-amino]-1,3,5-triazin-2,4-diyl)diimino]bis(benzoesäure-2-ethylhexylester) (Uvasorb HEB) oder 2,4-Bis[4-(2-ethylhexyloxy)-2-hydroxyphenyl]-6-(4-methoxyphenyl)-1,3,5-triazin (Tinosorb S).

Die erfindungsgemäßen O/W-Emulsionen umfassen grundsätzlich eine Ölphase, Wasser, ggf. Alkohol sowie die erfindungsgemäße Wirkstoffkombination. Die Ölphase kann dabei aus Fetten, Ölen, gelösten Wachsen oder sonstigen lipophilen Bestandteilen gebildet werden, insbesondere auch öllöslichen UV-Filtern. Als öle können vorteilhaft Paraffinöl, mittelkettige Triglyceride, wie beispielsweise Myritol 318, Octyldodecanol, Isopropylmyristat, Jojobaöl oder Kokosnussöl enthalten sein. Die wässrige Phase der erfindungsgemäßen Emulsionen wird üblicherweise aus Wasser, ggf. im Gemisch mit Alkohol gebildet.

Besonders bevorzugt sind die erfindungsgemäßen Zusammensetzungen frei von die Hautverträglichkeit potentiell negativ beeinflussenden Zusatzstoffen, insbesondere enthalten sie keine PEG-Emulgatoren, Konservierungsstoffe oder Duftstoffe, Vorzugsweise sind die Zubereitungen wasserfest.

Das folgende Ausführungsbeispiel soll die vorliegende Erfindung veranschaulichen, ohne sie zu beschränken. Die Inhaltsstoffe sind gemäß INCI-Nomenklatur bezeichnet worden.

Beispiel 1: O/W-Emulsion mit einem Lichtschutzfaktor ≥ 50

| INCI | g/100 g |
| --- | --- |
| Cetyl Alcohol | 1,00 |
| Cetearyl Alcohol | 1,00 |
| Ethylhexyl Methoxycinnamate | 10,00 |
| Diethylamino Hydroxybenzoyl Hexyl Benzoate | 7,00 |
| Octyldodecanol | 8,00 |
| Decyl Glucoside | 1,00 |
| Zinc Oxide | 0,60 |
| Titanium Dioxide, Aluminium Hydroxide Stearic Acid | 8,00 |
| Methylene Bis-Benzotriazolyl Tetramethylbutylphenol | 7,50 |
| Xanthan Gum | 0,30 |
| Acrylates/ C10-30 Alkyl Acrylate Crosspolymer | 0,50 |
| Sodium EDTA Disadium | 0,10 |

(fortgesetzt)

| INCI | g/100 g |
|------|---------|
| Glycerin | 5,00 |
| Panthenol | 1,00 |
| Alcohol Denat. | 10,00 |
| Sodium Hydroxide | 0,05 |
| Aqua | 38,95 |

Die O/W-Emulsion gemäß des Ausführungsbeispiels wird wie folgt hergestellt:

In einer Phase I werden Cetyl Alcohol, Cetearyl Alcohol, Ethylhexyl Methoxycinnamate, Diethylamino Hydroxybenzoyl Hexyl Benzoate und Octyldodecanol zusammengegeben, bei 80 °C unter Rühren von 25 Umdrehungen/Minute geschmolzen und die Temperatur 15 Minuten gehalten.

Dann werden Zinkoxide und Titandioxid zugegeben und unter Rühren dispergiert.

In einem separaten Gefäß wird eine Phase II, bestehend aus Methylen Bis-Benzotriazolyl Tetramethylbutylphenol und Decyl Glucoside gemischt.

In einem Wasserphasenkessel wird als Phase III Wasser vorgelegt und Glycerin, Dexpanthenol sowie Natrium EDTA Dinatrium zugegeben und unter 5 minütigem Rühren mit 25 Umdrehungen pro Minute gelöst. Das Xanthan Gum wird aufgestreut und dispergiert. Es wird anschließend für 10 Minuten homogenisiert. Anschließend wird das Acrylates/C10-30 Alkyl Acrylate Crosspolymer aufgestreut und dispergiert sowie für weitere 10 Minuten homogenisiert.

In einem separaten Gefäß wird als Phase IV Wasser und Natriumhydroxid gelöst.

In einem weiteren separaten Gefäß wird als Phase V vergällter Alkohol bereitgestellt.

Die Phase IV wird langsam unter Rühren zur Phase III in den Ansatzkessel gegeben und 10 Minuten mit 30 Umdrehungen/Minute gerührt.

Danach wird die Phase II langsam unter Rühren in den Ansatzkessel gegeben, und 3 Minuten mit einem Umlauf auf Stufe 2 unter Rühren mit 30 Umdrehungen/Minute homogenisiert.

Anschließend wird die Phase I langsam unter Homogenisieren in den Ansatzkessel gesaugt und weitere 10 Minuten mit Umlaufstufe 2 bei 30 Umdrehungen/Minute unter Kühlen homogenisiert.

Dann wird die Phase V langsam unter Rühren in den Ansatzkessel gesaugt und weitere 25 Minuten mit Umlaufstufe 2 bei 30 Umdrehungen/Minute unter Kühlung homogenisiert.

## Patentansprüche

1. Lichtschutzzubereitung in Form einer O/W-Emulsion,
   **dadurch gekennzeichnet, dass** sie die pigmentären Lichtschutzfilter Titandioxid, Zinkoxid und Methylene Bis-Benzotriazolyl Tetramethylbutylphenol enthält und einen Lichtschutzfaktor von $\geq 50$ aufweist, wobei das Titandioxid in Mengen von 3 bis 12 Gew.-%, das Zinkoxid in Mengen von 0,2 bis 2 Gew.-% und das Methylene Bis-Benzotriazolyl Tetramethylbutylphenol in Mengen von 5 bis 10 Gew.-% enthalten ist, und wobei die Zubereitung mindestens einen weiteren UV-A-Filter enthält.

2. Lichtschutzzubereitung gemäß Anspruch 1,
   **dadurch gekennzeichnet, dass** sie frei von PEG-Emulgatoren ist.

3. Lichtschutzzubereitung gemäß Anspruch 1 oder 2,
   **dadurch gekennzeichnet, dass** der UV-A-Filter Butyl Methoxydibenzoylmethane ist.

4. Lichtschutzzubereitung gemäß Anspruch 1 oder 2,
   **dadurch gekennzeichnet, dass** der UV-A-Filter Diethylamino Hydroxybenzoyl Hexyl Benzoate ist.

5. Lichtschutzzubereitung gemäß einem der Ansprüche 1 bis 4,
   **dadurch gekennzeichnet, dass** der UV-A-Filter in Mengen von 0,5 bis 8 Gew.-% enthalten ist.

6.  Lichtschutzzubereitung gemäß einem der Ansprüche 1 bis 5,
    **dadurch gekennzeichnet, dass** sie zusätzlich mindestens einen weiteren organischen Lichtschutzfilter enthält,
    vorzugsweise das Ethylhexyl Methoxycinnamate.
    VH:SG:sm

7.  Lichtschutzzubereitung gemäß einem der Ansprüche 1 bis 6,
    **dadurch gekennzeichnet, dass** das Titandioxid eine mittlere Teilchengröße von
    < 100 μm, bevorzugt eine Teilchengröße zwischen 10 nm und 200 nm und besonders bevorzugt eine Teilchengröße
    zwischen 10 nm und 100 nm aufweist.

8.  Lichtschutzzubereitung gemäß einem der Ansprüche 1 bis 7,
    **dadurch gekennzeichnet, dass** die Zinkoxidpigmente eine mittlere Teilchengröße
    < 100 μm, besonders bevorzugt eine mittlere Teilchengröße < 300 nm aufweisen.

9.  Lichtschutzzubereitung gemäß einem der Ansprüche 1 bis 8,
    **dadurch gekennzeichnet, dass** das Methylene Bis-Benzotriazolyl Tetramethylbutylphenol eine mittlere Teilchen-
    größe von < 100 μm, insbesondere 0,2 μm aufweist.

10. Lichtschutzzubereitung gemäß einem der Ansprüche 1 bis 9,
    **dadurch gekennzeichnet, dass** das Verhältnis der anorganischen zu den organischen Pigmenten im Bereich von
    0,2 bis 5, besonders bevorzugt von 0,5 bis 2,0 liegt.

11. Lichtschutzzubereitung gemäß einem der Ansprüche 1 bis 10,
    **dadurch gekennzeichnet, dass** das Verhältnis der Zinkoxidpigmente zu den Titandioxidpigmente im Bereich von
    0,01 bis 1,0, besonders bevorzugt von 0,05 bis 0,5 liegt.

12. Lichtschutzzubereitung gemäß einem der Ansprüche 1 bis 11,
    **dadurch gekennzeichnet, dass** das Zinkoxid eine mittlere Teilchengröße von < 50 nm aufweist, das Titandioxid
    eine mittlere Teilchengröße von 10-15 nm aufweist und das Methylene Bis-Benzotriazoyl Tetramethylbutylphenol
    eine mittlere Teilchengröße von 0,2 μm aufweist.

13. Lichtschutzzubereitung gemäß Anspruch 12, wobei das Zinkoxid in Mengen von 0,5-0,7 Gew.-%, das Titandioxid
    in Mengen von 7-9 Gew.-% und das Methylene Bis-Benzotriazolyl Tetramethylbutylphenol in Mengen von 6-9 Gew.-
    %, insbesondere 7,5 Gew.-% vorliegt.

14. Lichtschutzzubereitung gemäß einem der Ansprüche 1 bis 13,
    **dadurch gekennzeichnet, dass** sie frei von Konservierungsstoffen ist.

15. Lichtschutzzubereitung gemäß einem der Ansprüche 1 bis 14,
    **dadurch gekennzeichnet, dass** sie frei von Parfümöle ist.


## Claims

1.  A light protection preparation in the form of an o/w emulsion, **characterised in that** it contains the pigmentary light
    protection filters titanium dioxide, zinc oxide and methylene bis-benzotriazolyl tetramethylbutylphenol and has a
    light protection factor of ≥ 50, in which is contained the titanium dioxide in amounts of 3 to 12 wt.%, the zinc oxide
    in amounts of 0.2 to 2 wt.% and the methylene bis-benzotriazolyl tetramethylbutylphenol in amounts of 5 to 10 wt.%,
    and in which the preparation contains at least one more UV-A filter.

2.  A light protection preparation as claimed in claim 1, **characterised in that** it is free of PEG emulsifiers.

3.  A light protection preparation as claimed in claim 1 or claim 2, **characterised in that** the UV-A filter is butyl methoxydibenzoylmethane.

4.  A light protection preparation as claimed in claim 1 or claim 2, **characterised in that** the UV-A filter is diethylamino
    hydroxybenzoyl hexyl benzoate.

**5.** A light protection preparation as claimed in one of claims 1 to 4, **characterised in that** the UV-A filter is contained in amounts of 0.5 to 8 wt.%.

**6.** A light protection preparation as claimed in one of claims 1 to 5, **characterised in that** it contains in addition at least one more organic light protection filter, preferably ethylhexyl methoxycinnamate.

**7.** A light protection preparation as claimed in one of claims 1 to 6, **characterised in that** the titanium dioxide has an average particle size of < 100 $\mu$m, preferably a particle size between 10 nm and 200 nm, and a particularly preferred particle size between 10 nm and 100 $\mu$m.

**8.** A light protection preparation as claimed in one of claims 1 to 7, **characterised in that** the zinc oxide pigment has an average particle size of < 100 $\mu$m and a particularly preferred average particle size of < 300 $\mu$m.

**9.** A light protection preparation as claimed in one of claims 1 to 8, **characterised in that** the methylene bis-benzotriazolyl tetramethylbutylphenol has an average particle size of < 100 $\mu$m and in particular 0.2 $\mu$m.

**10.** A light protection preparation as claimed in one of claims 1 to 9, **characterised in that** the ratio of inorganic to organic pigments is in the region of 0.2 to 5, and particularly preferred in the region of 0.5 to 2.0.

**11.** A light protection preparation as claimed in one of claims 1 to 10, **characterised in that** the ratio of zinc oxide pigments to titanium dioxide pigments is in the region of 0.01 to 1.0, and particularly preferred in the region of 0.05 to 0.5.

**12.** A light protection preparation as claimed in one of claims 1 to 11, **characterised in that** the zinc oxide has an average particle size of < 50 nm, the titanium dioxide has an average particle size of 10-15 nm, and the methylene bis-benzotriazolyl tetramethylbutylphenol has an average particle size of 0.2 $\mu$m.

**13.** A light protection preparation as claimed in claim 12, in which the zinc oxide is present in amounts of 0.5-0.7 wt.%, the titanium dioxide is present in amounts of 7-9 wt.%, and the methylene bis-benzotriazolyl tetramethylbutylphenol is present in amounts of 6-9 wt.%, in particular 7.5 wt.%.

**14.** A light protection preparation as claimed in one of claims 1 to 13, **characterised in that** it is free of preservatives.

**15.** A light protection preparation as claimed in one of claims 1 to 14, **characterised in that** it is free of perfume oils.

**Revendications**

**1.** Préparation de protection contre la lumière sous la forme d'une émulsion huile/eau, **caractérisée en ce qu'**elle contient les filtres pigmentaires de protection contre la lumière dioxyde de titane, oxyde de zinc et méthylène bis-benzotriazolyl tétraméthylbutylphénol et présente un facteur de protection contre la lumière de $\geq$ 50, dans laquelle le dioxyde de titane est contenu en des quantités de 3 à 12 % en poids, l'oxyde de zinc en des quantités de 0,2 à 2 % en poids et le méthylène bis-benzotriazolyl tétraméthylbutylphénol en des quantités de 5 à 10 % en poids, et dans laquelle la préparation contient au moins un autre filtre UV-A.

**2.** Préparation de protection contre la lumière selon la revendication 1, **caractérisée en ce qu'**elle est exempte d'émulsifiants PEG.

**3.** Préparation de protection contre la lumière selon la revendication 1 ou 2, **caractérisée en ce que** le filtre UV-A est le butyl méthoxydibenzoylméthane.

**4.** Préparation de protection contre la lumière selon la revendication 1 ou 2, **caractérisée en ce que** le filtre UV-A est le diéthylamino hydroxybenzoyl hexyle benzoate.

**5.** Préparation de protection contre la lumière selon l'une quelconque des revendications 1 à 4, **caractérisée en ce que** le filtre UV-A est contenu en des quantités de 0,5 à 8 % en poids.

**6.** Préparation de protection contre la lumière selon l'une quelconque des revendications 1 à 5, **caractérisée en ce**

**qu'**elle contient en plus au moins un autre filtre organique de protection contre la lumière, de préférence le éthylhexyle méthoxycinnamate.

7. Préparation de protection contre la lumière selon l'une quelconque des revendications 1 à 6, **caractérisée en ce que** le dioxyde de titane présente une taille moyenne de particules de < 100 $\mu$m, de préférence une taille de particules comprise entre 10 nm et 200 nm et de préférence encore une taille de particules comprise entre 10 nm et 100 nm.

8. Préparation de protection contre la lumière selon l'une quelconque des revendications 1 à 7, **caractérisée en ce que** les pigments d'oxyde de zinc présentent une taille moyenne de particules < 100 $\mu$m, de préférence une taille moyenne de particules < 300 nm.

9. Préparation de protection contre la lumière selon l'une quelconque des revendications 1 à 8, **caractérisée en ce que** le méthylène bis-benzotriazolyl tétraméthylbutylphénol présente une taille moyenne de particules de < 100 $\mu$m, en particulier de 0,2 $\mu$m.

10. Préparation de protection contre la lumière selon l'une quelconque des revendications 1 à 9, **caractérisée en ce que** le rapport des pigments inorganiques aux pigments organiques se situe dans la plage de 0,2 à 5, de préférence de 0,5 à 2,0.

11. Préparation de protection contre la lumière selon l'une quelconque des revendications 1 à 10, **caractérisée en ce que** le rapport des pigments d'oxyde de zinc aux pigments de dioxyde de titane se situe dans la plage de 0,01 à 1,0, de préférence de 0,05 à 0,5.

12. Préparation de protection contre la lumière selon l'une quelconque des revendications 1 à 11, **caractérisée en ce que** l'oxyde de zinc présente une taille moyenne de particules de < 50 nm, le dioxyde de titane présente une taille moyenne de particules de 10-15 nm et le méthylène bis-benzotriazolyl tétraméthylbutylphénol présente une taille moyenne de particules de 0,2 $\mu$m.

13. Préparation de protection contre la lumière selon la revendication 12, dans laquelle l'oxyde de zinc est présent en des quantités de 0,5-0,7 % en poids, le dioxyde de titane est présent en des quantités de 7-9 % en poids, et le méthylène bis-benzotriazolyl tétraméthylbutylphénol est présent en des quantités de 6-9 % en poids, en particulier de 7,5 % en poids.

14. Préparation de protection contre la lumière selon l'une quelconque des revendications 1 à 13, **caractérisée en ce qu'**elle est exempte de substances de conservation.

15. Préparation de protection contre la lumière selon l'une quelconque des revendications 1 à 14, **caractérisée en ce qu'**elle est exempte d'huiles parfumées.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- *International Sun Protection Factor Method,* 2006